# EUROPÄISCHE PATENTANMELDUNG

(11) **EP 3 473 197 A1**
(43) Veröffentlichungstag der Anmeldung: **24.04.2019**
(21) Anmeldenummer: 17196972.8
(22) Anmeldetag: 18.10.2017
(51) Int. Cl.: A61B 17/3207, A61B 17/22, A61M 25/10

(54) **BALLONKATHETER**

(71) Anmelder: Biotronik AG, 8180 Bülach (CH)
(72) Erfinder: Schäfer, Tobias, 78176 Blumberg-Fützen (DE); Risch, Fabian, 8200 Schaffhausen (CH)
(74) Vertreter: Galander, Marcus

(57) **Zusammenfassung**

Ballonkatheter mit einem Katheterschaft (3a) und einem aufweitbaren Ballonabschnitt (3c), sowie mit Bearbeitungsmitteln zur Bearbeitung von Gefäßablagerungen,
wobei die Bearbeitungsmittel mindestens einen auf dem Umfang des Ballonabschnitts verlegten, proximal offenen und distal verschlossenen Aufsatzschlauch (5) aufweisen.

## Beschreibung

Die Erfindung betrifft einen Ballonkatheter mit mindestens einem Katheterschaft und einem aufweitbaren Ballonabschnitt, sowie mit Bearbeitungsmitteln zur Bearbeitung von Gefäßablagerungen.

Ballonkatheter und Ballonkatheter-Stent-Vorrichtungen sind seit langem bekannt und hunderttausendfach im klinischen Einsatz zur Behandlung von Gefäßverengungen (Stenosen) von Blutgefäßen. Die seit langem bekannte Behandlung durch Aufweitung (Dilatation) mittels eines Ballonkatheters wird bei solchen Vorrichtungen ergänzt um eine Stabilisierung des aufgeweiteten Gefäßabschnitts mittels eines Stützelementes, das durch den mittels des Ballons aufgeweiteten und nach der Deflatation des Ballons und dem Zurückziehen des Ballonkatheters am Ort der Stenose zurückgelassenen Stent (Stenting). Zu derartigen Vorrichtungen wird rein beispielhaft auf die DE 10 2004 059 523 A1 hingewiesen, die eine neuere Entwicklungsstufe dieser Vorrichtungen beschreibt.

In vielen Fällen ist zusätzlich zur Aufweitung und Stabilisierung des Stenose-Abschnitts ein mindestens partielles Abtragen von Ablagerungen auf der Gefäßwand wünschenswert. Zu diesem Zweck wurden Ballonkatheter mit sogenanntem Schneidballon (Cutting Balloons) mit Schneidklingen auf der Ballonoberfläche sowie neuerdings auch Stents mit Werkzeugelementen (Scoring Elements) zur Bearbeitung von Gefäßablagerungen entwickelt und auch bereits klinisch eingesetzt.

Die WO 2009/046206 A1 zeigt einen Ballonkatheter mit aufgesetzten Scoring-Elementen. Es handelt sich hierbei um auf den Ballonabschnitt des Ballonkatheters aufgesetzte, helikal verlaufende Metalldrähte oder vergleichbare Kunststoffelemente. Die US 8,348,987 B2 zeigt eine Ballonkatheter-Stent-Vorrichtung mit auf der Stentstruktur aufgesetzten Scoring-Elementen.

Es hat sich gezeigt, dass beide Lösungen erhebliche Nachteile haben.

Schneidballone sind in Folge der aufgesetzten Schneidelemente relativ steif und können nur schwer in komplexe Läsionen eingeführt werden, und es besteht hier auch ein nennenswertes Perforationsrisiko. Über dies ist der minimale (deflatierte) Ballondurchmesser größer als bei vergleichbaren Ballonen ohne Schneidelemente. Schließlich leisten Schneidballone beim Vorschieben der Vorrichtung durch das Gefäßsystem einen relativ großen Widerstand, der die präzise Handhabung erschwert.

Bei Stents mit Scoring-Elementen besteht unter Anderem die Gefahr, dass die auch im Einführungs-Zustand abstehenden Scoring-Elemente an unerwünschten und unvorhergesehenen Stellen zu Gewebeschädigungen führen. Andererseits sind die am beabsichtigten Einsatzort bei klinischen Studien tatsächlich erzielten Wirkungen nicht zufriedenstellend.

Der Erfindung liegt daher die Aufgabe zu Grunde, einen verbesserten Ballonkatheter der eingangs genannten Art bereitzustellen, der leicht und sicher handhabbar und flexibel entsprechend unterschiedlichen Anwenderbedürfnissen konfigurierbar ist.

Die Erfindung schließt den Gedanken ein, den Ballonkatheter mit Bearbeitungsmitteln auszustatten, die im Ausgangs-Zustand mit deflatiertem Ballon, also während des Einführens des Ballonkatheters, eine andere physikalische Beschaffenheit haben als im End-Zustand mit inflatiertem Ballon, in dem sie für eine Bearbeitung einer Stenose geeignet sein sollen. Insbesondere sollen sie im Ausgangs-Zustand eine geringe Steifigkeit haben und auch möglichst wenig über die Umfangsfläche des (noch nicht inflatierten) Ballonabschnitts hervorstehen, während sie im End-Zustand in für eine Bearbeitung hinreichendem Maße über die Ballonoberfläche vorstehen und ausreichend steif sein sollen.

Die Erfindung schließt weiter den Gedanken ein, diesen "Zustandsübergang" in analoger Weise zu bewirken wie beim Katheterballon selbst, nämlich durch Füllen mit einer geeigneten Flüssigkeit. Schließlich mündet die Erfindung in den Gedanken, dass die Bearbeitungsmittel mindestens einen mit geeignetem Verlauf auf den Ballonabschnitt des Katheters aufgesetzten zusätzlichen Schlauch mit verschlossenem Ende aufweisen, der nachfolgend als Aufsatzschlauch bezeichnet wird.

Unter einem Ballonkatheter wird im Rahmen dieser Anmeldung ein Katheter verstanden, welcher geeignet ist in ein Körpergefäß bzw. Lumen eines Patienten, insbesondere in ein Lumen des Blutkreislaufes, eingeführt zu werden. Ein derartiger Ballonkatheter umfasst mindestens einen Katheterschaft und einen aufweitbaren Ballonabschnitt, kurz Ballon. Die Aufweitung (Inflation) des Ballon erfolgt über die Beaufschlagung mit einem Fluid über ein Lumen, des Katheterschaftes.

In zweckmäßigen Ausführungen der Erfindung ist eine Mehrzahl von derartigen Aufsatzschläuchen, insbesondere 3 bis 5 Aufsatzschläuchen, vorgesehen. Hierdurch lassen sich bessere Bearbeitungseffekte als mit einem einzelnen Aufsatzschlauch realisieren.

Insbesondere ist der Aufsatzschlauch oder sind die Aufsatzschläuche helikal auf dem Ballonabschnitt verlegt, wobei sie zwischen dem proximalen Ballonhals und dem distalen Ballonhals insbesondere mindestens eine halbe Windung um die Längsachse des Ballonabschnitts beschreiben. Welchen Winkelbereich eines helikalen Verlaufs zwischen proximalem und distalem Ballonhals der Aufsatzschlauch oder die Aufsatzschläuche haben, wird der Fachmann entsprechend der konkreten Konfiguration, insbesondere im Hinblick auf die Länge des Ballonabschnitts, die Anzahl der aufgesetzten Schläuche und Anforderungen des Anwendungsfalls, bestimmen. Insbesondere wenn mehrere Aufsatzschläuche vorgesehen sind, ist grundsätzlich auch eine zur Längsachse des Ballonabschnitts parallele Verlegung möglich.

In einer aus derzeitiger Sicht zweckmäßigen Realisierung der Erfindung ist der oder jeder Aufsatzschlauch mindestens über den größeren Teil seiner Länger auf den Ballonabschnitt aufgeschweißt oder aufgeklebt. Alternativ hierzu ist es möglich, dass der Aufsatzschlauch im Wesentlichen nur am proximalen Ballonhals mit dem Katheterschaft fest verbunden, insbesondere verschweißt oder verklebt, ist. Bei letzterer Variante erscheint es als vorteilhaft, wenn der oder jeder Aufsatzschlauch auch dazwischen punktuell auf den Ballonabschnitt aufgeschweißt oder aufgeklebt ist, um unerwünschte tangentiale und/oder axiale Verschiebungen des Schlauches oder der Schläuche bezüglich des Ballons und damit verbundene Verschlechterungen der Bearbeitungswirkung zu verhindern.

In einer weiteren Ausführung umfasst der Katheterschaft einen Innenschaft und einen Außenschaft, und es ist das offene proximale Ende des oder jedes Aufsatzschlauches mit dem Lumen des Außenschafts fluidmäßig verbunden. Der Aufsatzschlauch oder die Aufsatzschläuche werden hierbei also zugleich mit dem Katheterballon unter Druck gesetzt, was den Aufbau des Katheters insgesamt und auch dessen Handhabung besonders einfach gestaltet. In einer anderen, als solche bekannten Katheterkonstruktion weißt der Katheterschaft ein erstes Lumen für einen Führungsdraht und ein zweites Lumen zur Einleitung eines Fluids zur Aufweitung des Ballonabschnitts auf, und das offene proximale Ende des oder jedes Aufsatzschlauches ist mit dem zweiten Lumen fluidmäßig verbunden ist.

Alternativ hierzu ist vorgesehen, dass das offene proximale Ende des oder jedes Aufsatzschlauches in einen separaten proximalen Fluidanschluss des Ballonkatheters mündet. Dies bietet die Möglichkeit einer von der Inflation des Ballons unabhängigen Steuerung der Beschaffenheit der Bearbeitungsmittel, erfordert allerdings eine sich bis zum proximalen Katheterende erstreckende gesonderte Fluidleitung bzw. ein zusätzliches Lumen im Katherkörper und einen separaten Anschluss des Katheters.

Hierbei besteht zum Einen die Möglichkeit, dass das offene proximale Ende des oder jedes Aufsatzschlauches über ein zusätzliches Lumen im Katheterschaft mit dem dedizierten Fluidanschluss verbunden ist. Alternativ kann, als Modifikation bekannter Katheterkonfigurationen, das offene proximale Ende des oder jedes Außenschlauches über einen außen am Katheterschaft verlegten Bearbeitungsfluidschlauch mit dem gesonderten Fluidanschluss verbunden sein.

In einer weiteren Ausführung der Erfindung hat der Ballonkatheter eine längs des Katheterschaftes verschieblichen Bearbeitungsmittel-Schutzhülle. Diese Schutzhülle ist vor und während der Einführung des Katheters bis zum distalen Ballonhals vorgeschoben und deckt den Außenschlauch oder die Außenschläuche ab und wird nach der Einführung unter Freilegung des Außenschlauchs oder der Außenschläuche zur Realisierung seines/ihres Bearbeitungszwecks bis mindestens zum proximalen Ballonhals zurückgezogen. Es versteht sich, dass bei einer solchen Konstruktion ein zusätzliches Betätigungselement für die Bearbeitungsmittel-Schutzhülle benötigt wird und die Handhabung eines solchen Katheters etwas aufwendiger wird.

In zweckmäßigen Ausführungen weist der oder jeder Aufsatzschlauch ein Material aus der Gruppe Polyamide, Polyimide, Polyester, Polyether, Polyolefine und deren Co-Polymere auf. Besonders bevorzugt sind Materialien, die insoweit verträglich mit dem Material des Katheterballons sind, als ein leichtes Verschweißen oder Verkleben möglich ist.

In weiteren Ausführungen der Erfindung liegt der Durchmesser des oder jeden Aufsatzschlauches im Bereich zwischen 2% und 8%, bevorzugt zwischen 2% und 4%, des Durchmessers des Ballons. Die konkrete Festlegung der Aufsatzschlauch-Geometrie wird der Fachmann insbesondere unter Beachtung des Aufbaus des Ballonkatheters als solchen sowie der Anwendungssituation im Rahmen fachgemäßen Handelns treffen.

In weiteren Ausführungen ist vorgesehen, dass der Aufsatzschlauch oder mindestens ein Teil der Aufsatzschläuche eine Schneid- oder Sägekante zur abrasiven Bearbeitung von Gefäßablagerungen aufweist. Die Schneid- oder Sägekante(n) kann(können) dabei proximal, distal und/oder über die gesamte Länge mit dem Aufsatzschlauch verklebt oder verschweißt werden. Alternativ kann(können) Schneid- oder Sägekante(n) auch direkt mit dem Ausformen des Aufsatzschlauches erzeugt werden. Als Materialien für die Schneid-oder Sägekante eignen sich insbesondere Metalle (beispielsweise Edelstahl) oder Polymere wie Polyamide, Polyimide, Polyester, Polyether, Polyolefine und deren Co-Polymere. Der Durchmessers eines Aufsatzschlauches (bzw. dessen maximale radiale Ausdehnung) beträgt vorteilhafterweise zwischen 4% und 8% des Ballondurchmessers. Dies ist insbesondere für Anwendungen vorteilhaft, wo der Ballonkatheter nicht nur ein Aufsprengen kalkartiger Ablagerungen, sondern auch ein Zertrennen und/oder schichtweises Abtragen leisten soll. Es versteht sich, dass das Vorsehen von Schneid- oder Sägekanten die Handhabung des Ballonkatheters bei der Einführung etwas erschweren kann; dieser Nachteil wird jedoch in bestimmten Anwendungssituationen von den Vorteilen einer derartigen Ausgestaltung überwogen.

Mit der Erfindung lassen sich zumindest in bestimmten Ausführungen unter anderem folgende Vorteile erzielen:
Vorteile und Zweckmäßigkeiten der Erfindung ergeben sich im Übrigen aus der nachfolgenden Beschreibung eines Ausführungsbeispiels anhand der Figuren. Von diesen zeigen:
   - Fig. 1: eine schematische perspektivische Darstellung eines Ballonkatheters gemäß einer ersten Ausführungsform der Erfindung,
   - Fig. 2: eine schematische perspektivische Darstellung eines Ballonkatheters gemäß einer weiteren Ausführungsform der Erfindung,
   - Fig. 3: eine schematische perspektivische Darstellung eines Ballonkatheters gemäß einer weiteren Ausführungsform der Erfindung,
   - Fig. 4: eine schematische Längsschnittdarstellung eines ersten Ballonkatheter-Grundaufbaus,
   - Fig. 5: eine schematische Längsschnittdarstellung eines zweiten Ballonkatheter-Grundaufbaus,
   - Fig. 6: eine schematische Längsschnittdarstellung eines Ballonkatheters gemäß einer weiteren Ausführungsform der Erfindung.

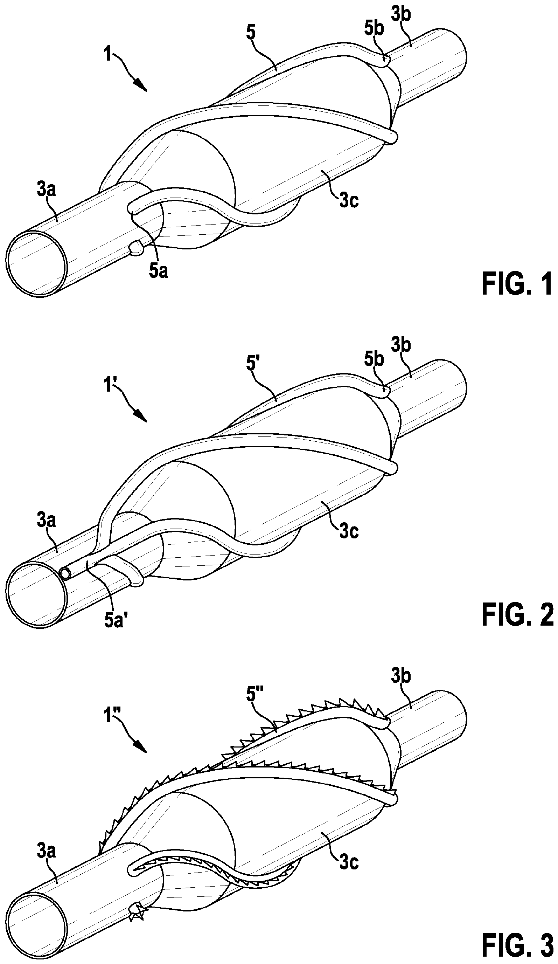

Fig. 1 zeigt skizzenartig einen Ballonkatheter 1, der einen Außenschaft 3a, einen Innenschaft 3b und einen (in der Figur inflatiert dargestellten) Ballonabschnitt 3c sowie drei auf dem Ballonanbschnitt 3c verlegte Aufsatzschläuche 5 aufweist. Die helikal in gleichen Winkelabständen über die Umfangsfläche des Ballonabschnitts 3c verlegten Aufsatzschläuche 5 haben jeweils ein proximal offenes Ende. Sie sind dort an Einmündungen 5a fluidmäßig mit dem Innenlumen des Katheterschafts 3a verbunden, werden also zugleich mit der Inflation des Ballons 3c und in gleicher Weise wie der Ballonabschnitt unter Fluiddruck gesetzt. An ihren distalen Enden 5b sind die Aufsatzschläuche 5 fluiddicht verschlossen. Die Aufsatzschläuche können über ihre gesamte Fläche oder auch nur punktuell, beispielsweise an den Halsabschnitten des Ballons 3c, mit dem Ballon 3c und/oder den angrenzenden Schaftabschnitten verschweißt, verklebt oder sonst wie fest verbunden sein.

Fig. 2 zeigt eine Modifikation des in Fig. 1 gezeigten Aufbaus, wobei für übereinstimmende oder funktional gleichartige Teile die gleichen Bezugsziffern vergeben sind und jene Teile hier nicht weiter beschrieben werden. Der wesentliche Unterschied das Ballonkatheters 1' gegenüber dem Ballonkatheter 1 nach Fig. 2 besteht in einer veränderten Ausführung der Aufsatzschläuche 5' an deren proximalem Ende. Hier münden diese nämlich nicht in den Katheterschaft 3a, sondern sind über ein Verzweigungsstück 5a' miteinander und mit einer zum proximalen Katheterende führenden (nicht gezeigten) separaten Fluidleitung verbunden. Bei dieser Ausführung werden die Aufsatzschläuche 5' unabhängig von der Inflation des Ballonabschnitts 3c über die separate Fluidleitung unter Fluiddruck gesetzt.

Fig. 3 zeigt einen weiteren Ballonkatheter 1", der durch eine Modifikation aus dem Ballonkatheter 1 nach Fig. 1 abgeleitet ist. Der wesentliche Unterschied besteht hier darin, dass auf den Aufsatzschläuchen 5" eine Sägezahnstruktur ausgebildet ist, die eine abrasive Bearbeitung von Gefäßablagerungen am Einsatzort des Ballonkatheters ermöglicht.

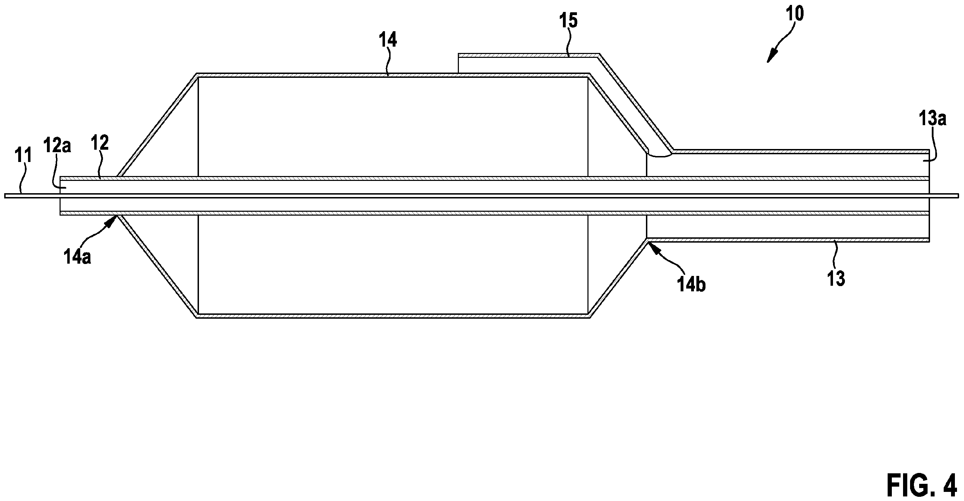

Fig. 4 zeigt schematisch in Art einer Längsschnittdarstellung eine erste Variante des Innenaufbaus eines erfindungsgemäßen Ballonkatheters 10 mit im Wesentlichen koaxialem Aufbau. Der (hier nicht gesondert bezeichnete) Katheterschaft umfasst einen Innenschaft 12, in dem ein Führungsdraht 11 aufgenommen ist, und einen Außenschaft 13. Ein Ballonabschnitt 14 ist an einem distalen Ballonhals 14a mit dem Innenschaft 12 und an einem proximalen Ballonhals 14b mit dem Außenschaft 13 verschweißt. Der Ballonabschnitt 14 ist hier im aufgeweiteten Zustand dargestellt. Zur Ausbildung dieses Zustandes ist im Außenschaft 13 ein Lumen 13a zur Zuführung eines Fluids in den Ballonabschnitt 14 vorgesehen, während ein zu dem Lumen 13a koaxiales Innenschaft-Lumen 12a im Innenschaft 12 den Führungsdraht 11 längsverschieblich aufnimmt. Ein Aufsatzschlauch 15 als Bearbeitungsmittel des Ballonkatheters 10 ist fluidmäßig am proximalen Ballonhals 14b mit dem Außenschaft-Lumen 13a verbunden. Der Aufsatzschlauch 15 wird somit zugleich mit dem Ballonabschnitt 14 inflatiert.

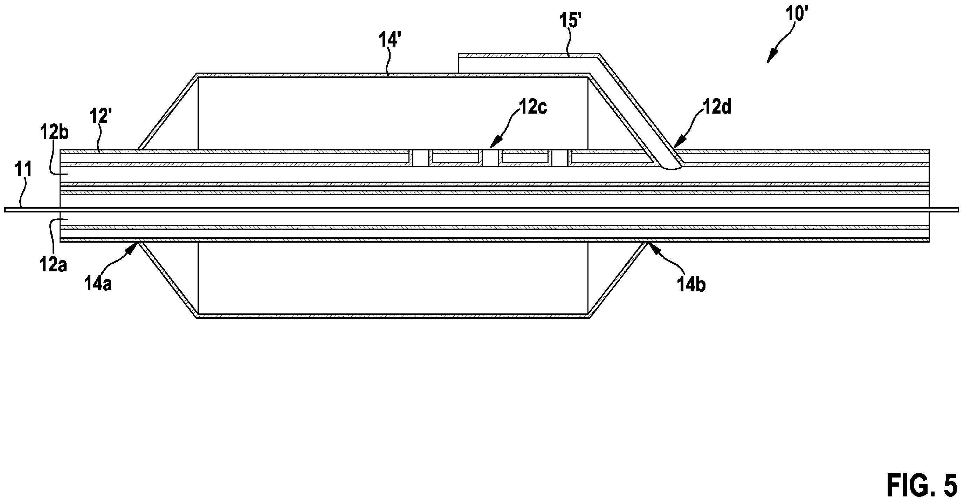

Fig. 5 zeigt einen weiteren Ballonkatheter 10' mit anderem Aufbau, wobei gleiche oder funktional vergleichbare Teile mit den gleichen Ziffern wie in Fig. 4 bezeichnet sind und hier nicht nochmals beschrieben werden. Im Unterschied zum Ballonkatheter 10 nach Fig. 4 hat der Ballonkatheter 10' einen einstückigen Katheterschaft 12', in dem parallel zueinander zwei Lumen 12a (für den Führungsdraht 11) und 12b (als Fluidkanal) eingearbeitet sind. Der Katheterschaft 12' ist sowohl am distalen Ballonhals 14a als auch am proximalen Ballonhals 14b mit dem Ballonabschnitt 14' verschweißt. Der Ballonabschnitt 14' wird über Durchgänge 12c vom Fluidkanal 12b in das Innere des Ballonabschnitts 14' mit einem vom proximalen Katheterende her zugeführten Fluid befüllt und hierdurch aufgeweitet. Ein weiterer Durchgang 12d verbindet den Fluidkanal 12b mit dem Aufsatzschlauch 15'. Fluid im Lumen 12b setzt den Aufsatzschlauch somit wiederum gleichzeitig mit dem Ballonabschnitt 14' unter Fluiddruck und weitet ihn auf. Alternativ zur gezeigten Konfiguration kann der Aufsatzschlauch 15' auch auf dem Katheterschaft 12' extern zum proximalen Katheterende verlaufen.

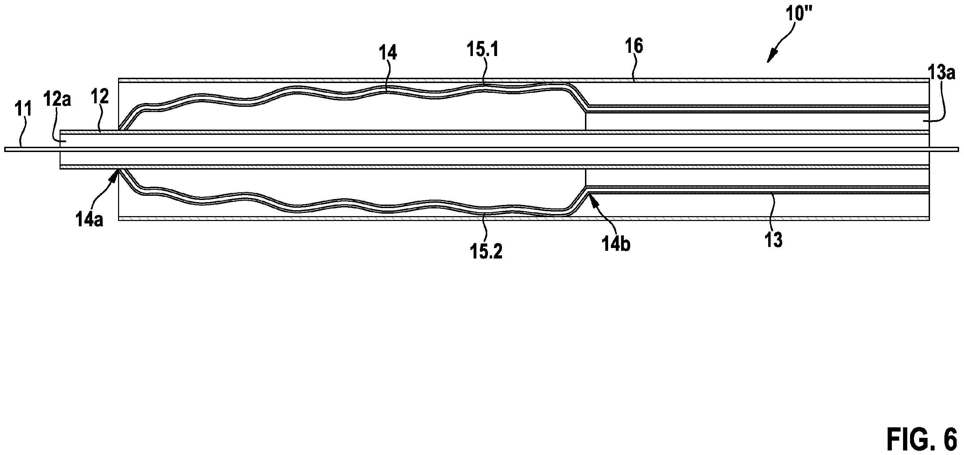

Eine weitere Alternative zu den in Fig. 4 und Fig. 5 gezeigten Konfigurationen besteht darin, dass innerhalb des Außenkatheters bei der Anordnung nach Fig. 4 oder innerhalb des Katheterschaftes bei der Anordnung nach Fig. 5 ein zusätzliches Lumen vorgesehen ist, welches ausschließlich der Fluidversorgung des Aufsatzschlauches oder der Aufsatzschläuche dient. Diese Variante ist in Fig. 6 schematisch dargestellt, und zwar zusammen mit einer weiteren Abwandlung der Konfiguration nach Fig. 4. Diese besteht darin, dass dem eigentlichen Ballonkatheter C2' eine Bearbeitungsmittel-Schutzhülle 16 zugeordnet ist. Diese überdeckt die Aufsatzschläuche 15.1 und 15.2 (sowie ggfs. weitere, hier nicht dargestellte) im Einführzustand und wird nach dem Einführen, bei Inbetriebnahme des Ballonkatheters mit seinen als Bearbeitungsmittel dienenden Aufsatzschläuchen proximal zurückgezogen.

Im Übrigen ist die Ausführung der Erfindung auch in einer Vielzahl von Abwandlungen der hier gezeigten Beispiele und weiter oben hervorgehobenen Aspekte der Erfindung möglich.

## Patentansprüche

1. Ballonkatheter (1, 1',1", 10; 10'; 10") mit einem Katheterschaft (12'; 12/13) und einem aufweitbaren Ballonabschnitt (3c; 14; 14'), sowie mit Bearbeitungsmitteln zur Bearbeitung von Gefäßablagerungen,
wobei die Bearbeitungsmittel mindestens einen auf dem Umfang des Ballonabschnitts verlegten, proximal offenen und distal verschlossenen Aufsatzschlauch (5; 5', 5"; 15; 15'; 15.1, 15.2) aufweisen.

2. Ballonkatheter nach Anspruch 1, wobei die Bearbeitungsmittel eine Mehrzahl von Aufsatzschläuchen (5; 5', 5"; 15; 15'; 15.1, 15.2), insbesondere drei bis fünf Aufsatzschläuche, umfassen.

3. Ballonkatheter nach Anspruch 1 oder 2, wobei der Aufsatzschlauch oder die Aufsatzschläuche (5; 5', 5"; 15; 15'; 15.1, 15.2) helikal auf dem Ballonabschnitt (3c 14; 14') verlegt ist/sind, wobei sie zwischen dem proximalen Ballonhals (14b) und dem distalen Ballonhals (14a) insbesondere mindestens eine halbe Windung um die Längsachse des Ballonabschnitts beschreiben.

4. Ballonkatheter nach einem der vorangehenden Ansprüche, wobei der oder jeder Aufsatzschlauch (5; 5', 5"; 15; 15'; 15.1, 15.2) mindestens über den größeren Teil seiner Länge auf den Ballonabschnitt aufgeschweißt oder aufgeklebt ist.

5. Ballonkatheter nach einem der Ansprüche 1 bis 3, wobei der oder jeder Aufsatzschlauch (5; 5', 5"; 15; 15'; 15.1, 15.2) im Wesentlichen nur am proximalen Ballonhals (14b) und am distalen Ballonhals (14a) am Katheterschaft (12'; 12/13) fest fixiert, insbesondere verschweißt oder verklebt, ist.

6. Ballonkatheter nach Anspruch 5, wobei der oder jeder Aufsatzschlauch (5; 5', 5"; 15; 15'; 15.1, 15.2) auch zwischen dem proximalen und distalen Ballonhals (14b, 14a) auf den Ballonabschnitt (3c; 14, 14') punktuell aufgeschweißt oder aufgeklebt ist.

7. Ballonkatheter nach einem der vorangehenden Ansprüche, wobei der Katheterschaft einen Innenschaft (3b; 12) und einen Außenschaft (3a; 13) umfasst, und das offene proximale Ende (5a) des oder jedes Aufsatzschlauches (5; 15) mit dem Lumen des Außenkatheters (3a; 13) fluidmäßig verbunden ist.

8. Ballonkatheter nach einem der Ansprüche 1 bis 6, wobei der Katheterschaft (12'; 12/13) ein erstes Lumen (12a) für einen Führungsdraht (11) und ein zweites Lumen (12b'; 13a) zur Einleitung eines Fluids zur Aufweitung des Ballonabschnitts aufweist und das offene proximale Ende des oder jedes Aufsatzschlauches mit dem zweiten Lumen (12b; 13a) fluidmäßig verbunden ist.

9. Ballonkatheter nach einem der Ansprüche 1 bis 6, wobei das offene proximale Ende (5a') des oder jedes Aufsatzschlauches (5'; 15.1, 15.2) in einen dedizierten proximalen Fluidanschluss des Ballonkatheters (1'; 10") mündet.

10. Ballonkatheter nach Anspruch 9, wobei das offene proximale Ende des oder jedes Aufsatzschlauches über ein zusätzliches Lumen im Katheterschaft mit dem dedizierten Fluidanschluss verbunden ist.

11. Ballonkatheter nach Anspruch 9, wobei das offene proximale Ende des oder jedes Aufsatzschlauches (15.1, 15.2) über ein außen am Katheterschaft (12/13) verlegten Bearbeitungsfluidschlauch mit dem dedizierten Fluidanschluss verbunden ist.

12. Ballonkatheter nach einem der vorangehenden Ansprüche, mit einer längs des Katheterschaftes (12/13) verschieblichen Bearbeitungsmittel-Schutzhülle (16).

13. Ballonkatheter nach einem der vorangehenden Ansprüche, wobei der oder jeder Aufsatzschlauch (5; 5', 5"; 15, 15'; 15.1, 15.2) ein Material aus der Gruppe Polyamide, Polyimide, Polyester, Polyether, Polyolefine und deren Co-Polymere aufweist.

14. Ballonkatheter nach einem der vorangehenden Ansprüche, wobei der Durchmesser des oder jeden Aufsatzschlauches (5; 5', 5"; 15, 15'; 15.1, 15.2) im Bereich zwischen 2% und 8%%, bevorzugt zwischen 2% und 4%, des Durchmessers des Ballons liegt

15. Ballonkatheter (1") nach einem der vorangehenden Ansprüche, wobei der Aufsatzschlauch oder mindestens ein Teil der Aufsatzschläuche (5") eine Schneid- oder Sägekante zur abrasiven Bearbeitung von Gefäßablagerungen aufweist.
